# EUROPÄISCHE PATENTSCHRIFT

(11) **EP 0 293 651 B1**
(45) Veröffentlichungstag und Bekanntmachung des Hinweises auf die Patenterteilung: **02.02.1994**
(21) Anmeldenummer: 88107614.5
(22) Anmeldetag: 11.05.1988
(51) Int. Cl.: C07D 307/54, C07D 405/04, C07D 413/04, C07D 417/04, C09K 9/02, B01J 31/02, G03C 1/10, G11B 7/24, C07D 317/54, G03C 1/73, H01B 1/12

(54) **Dicyanvinylsubstituierte Furanderivate, Verfahren zu ihrer Herstellung und deren Verwendung**
Dicyanovinyl-substituted furan derivatives, process for their preparation, and their use
Dérivés de furanne dicyanovinyle-substituée, leur procédé de préparation et leur utilisation

(30) Priorität: 05.06.1987 DE 3718917
(43) Veröffentlichungstag der Anmeldung: 07.12.1988
(73) Patentinhaber: SÜDZUCKER AKTIENGESELLSCHAFT MANNHEIM/OCHSENFURT, D-68165 Mannheim (DE)
(72) Erfinder: Daub, Jörg, Dr., D-8400 Regensburg (DE); Rapp, Knut, M., Dr., D-6521 Offstein (DE); Seitz, Petra, D-8440 Straubing (DE); Wild, Rainer, D-6719 Obrigheim 1 (DE); Salbeck, Josef Dr., D-8400 Regensburg (DE)
(74) Vertreter: Gleiss, Alf-Olav, Dr.jur. Dipl.-Ing.

(56) Entgegenhaltungen:
- CH-A- 390 050
- DE-A- 2 245 287
- GB-A- 2 142 011
- US-A- 4 184 871
- ANGEWANDTE CHEMIE, vol. 96, no. 12, 1984, Weinheim, DE, Seiten 980 - 981; J. DAUB et al.: "Lichtsensitive Dihydroazulene mit chromogenen Eigenschaften"

## Beschreibung

Die vorliegende Erfindung betrifft neuartige Furanderivate entsprechend der im Anspruch 1 wiedergegebenen allgemeinen Formel I, die sich aufgrund ihrer Eigenschaften in besonders günstiger Weise u.a. für elektro-optische Anwendungen eignen.

Stoffe, die durch Lichtabsorption unmittelbar zu einer Farbstoffbildung und damit einer Bilderzeugung fähig sind, also keine Entwicklungs- oder Waschprozesse benötigen, können in optischen Informationsspeicher- bzw. -verarbeitungssystemen verwendet werden. Da mit derartigen Substanzen kornlose Schichten herstellbar sind, sind sehr hohe Speicherdichten möglich (Chemiker-Zeitung 96, 535 (1972)).

Spiropyrane wie solche der Formel 1 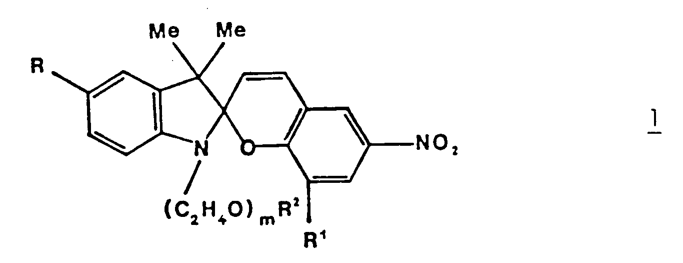
(vgl. JP 61 18 782 (86 18 782); CA. 105, 78 849 q (1986)) sind eine Substanzklasse, die durch UV-Belichtung in gefärbte Verbindungen übergehen und durch Wärmezufuhr wieder entfärbt werden können.

Die Anwendungen und Vorteile von reversiblen photochromen Stoffen sind in "Chemie in unserer Zeit" 9, 85 (1975) beschrieben, nämlich die hohe optische Auflösung (hohe Speicherdichte), die Kontrolle der Belichtung während des Schreibens, sowie die Möglichkeit der Löschung bzw. des Austauschens von Bildteilen.

In Kirk-Othmer, Encyclopedia of chemical technology 3. Ed. Vol. 6, 122 (1979) werden als Nachteile von organischen photochromen Materialien genannt: geringe Spektralverschiebung, langsame Rückreaktion, "Materialermüdung" sowie Kombinationen dieser Eigenschaften.

Als Substanzklassen, bei denen eine Valenztautomerie für das chromogene Verhalten verantwortlich ist, sind Spiropyrane sowie Fulgide erwähnt. Erstere können teilweise durch eine Copolymerisation mit einem geeigneten Monomer in ein Polymer eingebaut werden. Fulgide gehören ebenso zu den besser untersuchten photochromen Systemen (vgl. z.B. CA 102, 229 304z (1985), GB 2 142 011 zit. in CA 103, 79 543 f (1985) mit Verbindungen der Formel 2). 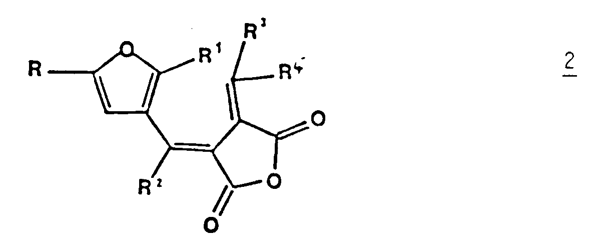
In Angew. Chem. 96, 980 (1984) wird ein System beschrieben, in dem ein Dihydroazulenderivat durch Lichtabsorption in ein gefärbtes Heptafulvenderivat überführt wird, das durch Zufuhr von Wärme wieder zum Dihydroazulen cyclisiert. Im Gegensatz zu R = NO₂ ist das System 3 A ≷ 3 B 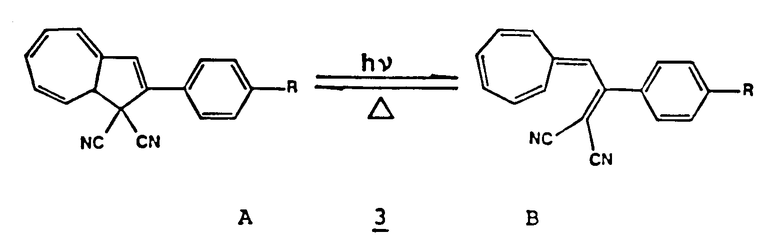
für R = OCH₃ chemisch stabil, d.h. bei mindestens 15-facher Hin- und Rückreaktion ändern sich die Extinktionswerte nicht, es finden also keine Neben- bzw. irreversiblen Abbaureaktionen statt.

Das oben dargestellte photochrome System wird synthetisiert nach folgender Reaktionsfolge: 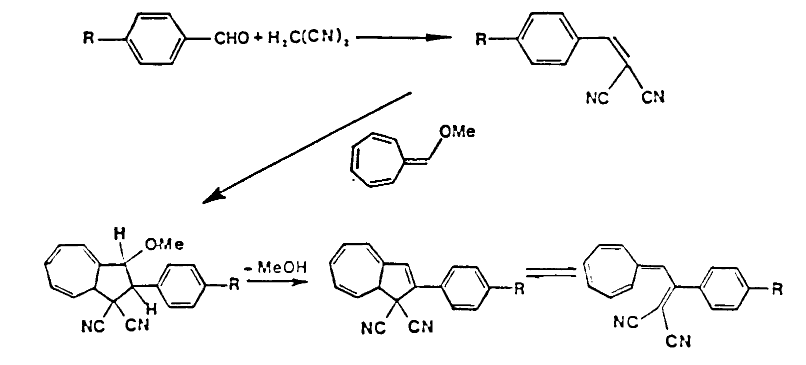
8-Methoxyheptafulven ist in zwei Stufen und guter Ausbeute aus Cyclooctatetraen zugänglich. Ein Nachteil des eingesetzten Anisaldehyds (R= OCH₃) ist das Fehlen einer funktionellen Gruppe, die es ermöglichen würde, das photochrome Produkt in geeigneter Weise z.B. kovalent mit Polymeren zu verknüpfen.

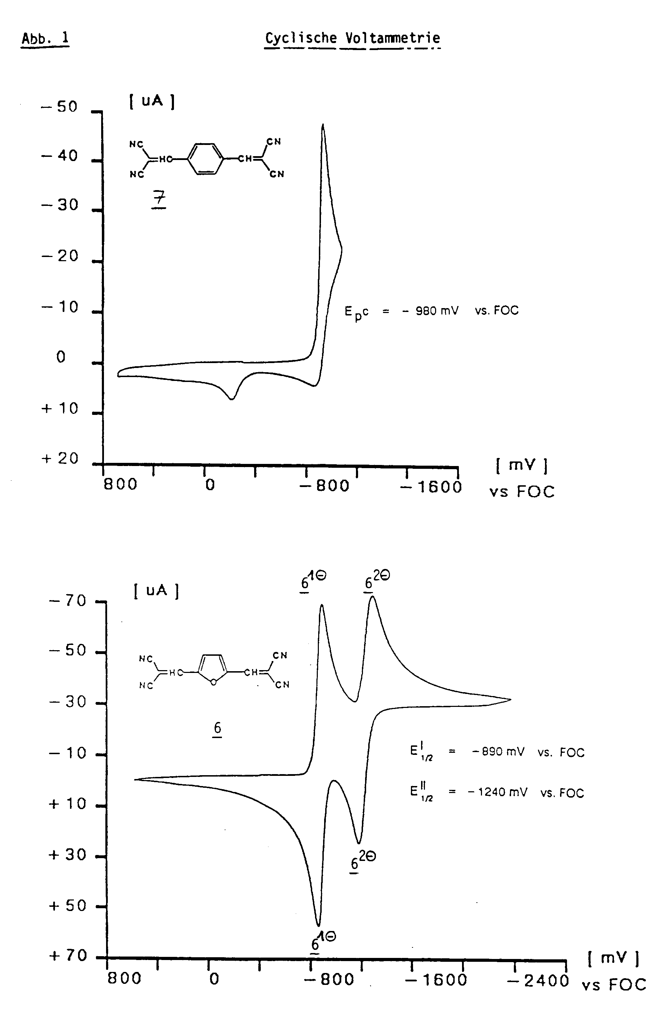

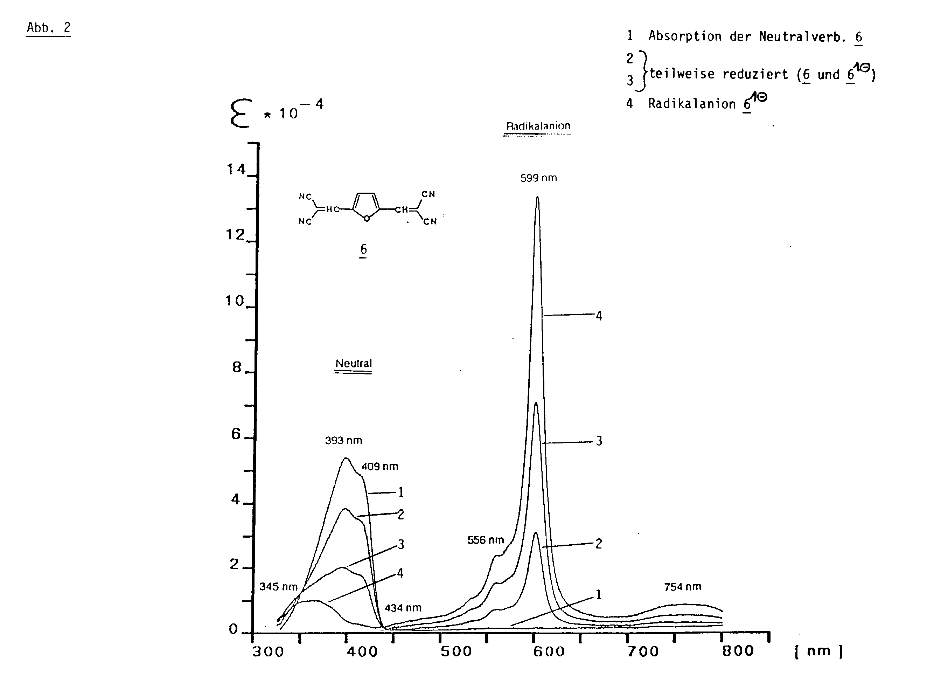

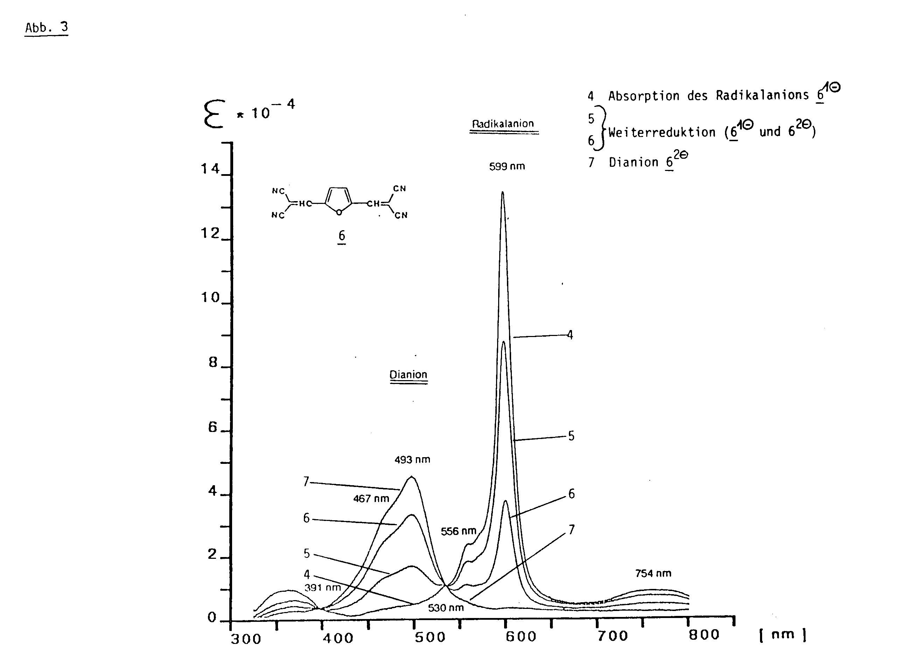

Überraschend vorteilhaft dagegen ist der Einsatz von 5-Hydroxymethylfurfural (im folgenden HMF genannt) der Formel 4 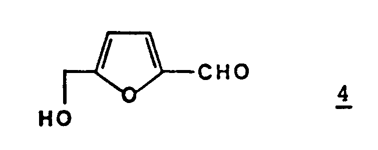
anstelle von Anisaldehyd aus folgenden Gründen:
1. Der elektronische EinfluB eines Furanringes auf gebundene Gruppen ist vergleichbar mit dem eines O-Alkyl-Benzolrings, das bedeutet, daß Furfural ähnlich reagiert wie Anisaldehyd (p-Methoxybenzaldehyd).
2. Die Hydroxymethylgruppe in HMF ermöglicht, als zusätzliche funktionelle Gruppe, eine Verknüpfung z.B. mit einem Oligo- oder Polymer. Eine derartige Verknüpfung wird in Kirk-Othmer, Encyclopedia of chemical technology 3. Ed. Vol. 6, 122 (1979) im Falle der Spiropyrane ausdrücklich als Vorteil erwähnt. Eine Derivatisierung an der Hydroxymethylgruppe erlaubt auch eine gezielte Beeinflussung der Löslichkeit.
3. Typische Carbonylreaktionen erlauben auch, durch eine Kondensation mit Aminen mit einer anschließenden Oxidation der Hydroxymethylgruppe zur Aldehydstufe, z.B. Verbindungen der Formel 5 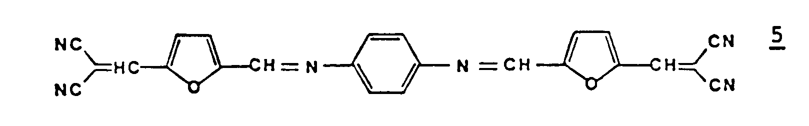 herzustellen, die über Knoevenagelreaktionen mit Malonsäuredinitril erhalten werden.
4. Durch die Möglichkeit, verschiedene "Brücken" zwischen 2 Furane einzubauen, ist eine Beeinflussung der Dicyanvinylgruppen gegeben und somit eine Variation der Elektronenakzeptoreigenschaften bzw. der di-oder polyenophilen Eigenschaften.
5. 5-Hydroxymethylfurfural hat als Ausgangsstoff noch den groBen Vorteil, in einem einzigen Reaktionsschritt aus nachwachsenden Rohstoffen (Kohlenhydraten) hergestellt werden zu können, und dies im großtechnischen Maßstab mit Wasser als einzigem Lösungsmittel. Insbesondere die Verwendung von landwirtschaftlichen, (zeitweise) im Überschuß produzierten Kohlenhydraten, wie z.B. Zucker (Saccharose) in technischen Gebieten über die Zwischenverbindung HMF zeigt den Vorteil von diesen erfindungsgemäßen dicyanvinylsubstituierten Furanen.
6. Ein weiterer überraschender Vorteil von Furylidenmalononitrilen gegenüber Phenylanalogen ist beispielsweise die volle Reversibilität der Reduktion der Verbindung gemäß der Formel 6, während die Verbindung gemäß der Formel 7 nicht reversibel reduzierbar ist. Dies ist auch im Zusammenhang zu sehen mit den in 1. aufgeführten Substituenteneffekten. 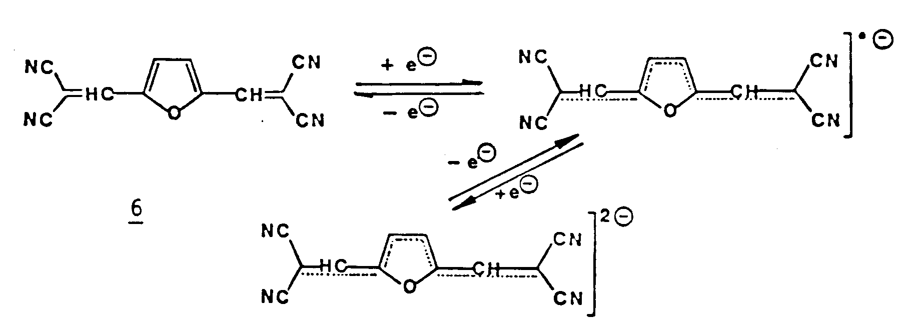 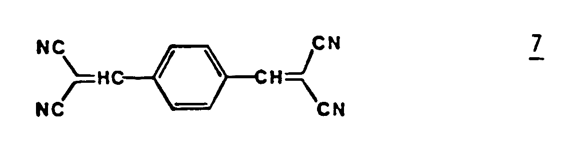 Das zeigt, daß die Verbindung 6 ähnlich wie Tetracyanochinodimethan (TCNQ) entsprechend der Formel 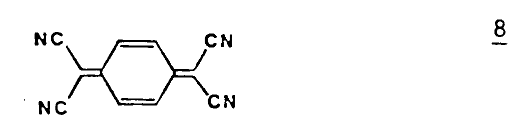 das ebenfalls in einer zweifachen Einelektronenreaktion reversibel reduziert werden kann, als Komponente von sog. "Organischen Metallen" verwendet werden kann, bzw. als Elektronenspeicher oder Elektronen-Transfer-Katalysator.
   Einzelheiten zu dem Verlauf der Reduktion von der Verbindung 6, bestimmt durch cyclische Voltametrie unter aprotischen Bedingungen und Leitsalzzusatz, unter Bildung des Radikalanions 6¹⁻ bzw. des Dianions 6²⁻ sowie bezüglich des Absorptionsverhaltens unter spektroelektrochemischen Gesichtspunkten bei Anwendung von optisch transparenten Elektroden und eines aprotischen Lösungsmittels ergeben sich aus den später folgenden Abbildungen 1 bis 3. Dem Radikalanion 6¹⁻ kommt danach eine außergewöhnlich schmale Absorptionsbande bei λ = 599 nm (dunkelblaue Lösung) mit hohem Extinktionskoeffizient zu. Im Gegensatz zu dem Radikalanion von dem vorgenannten TCNQ, also 8¹⁻ mit einer Absorption bei ca λ= 840 nm, absorbiert das Anion 6¹⁻ im Sichtbaren.
   Die reversible Reduzierbarkeit von Alkylidenmalononitrilen ist erwähnt in Angew. Chem. 88, 311 (1976). Die Bildung von Radikalanionen von Tetracyanoethylen oder TCNQ wird auch beschrieben in Kirk-Othmer, Encyclopedia of chemical technology 3. Ed. Vol. 7, 359, 362 (1979).
   Eine Korrelation zwischen reversiblen Redox-Eigenschaften (Elektrochemie) und chromogenen Eigenschaften (Photochemie) zeigt sich auch bei der länger bekannten Klasse der Fulgide. So ist in J. Amer.Chem.Soc. 106, 7626 (1984) beschrieben, daß das reversibel erzeugte Radikalanion von Formel 9 eine elektrocyklische Reaktion eingeht zu einer Struktur ähnlich der ringgeschlossenen, photochemisch erzeugten, von chromogenen Fulgiden. 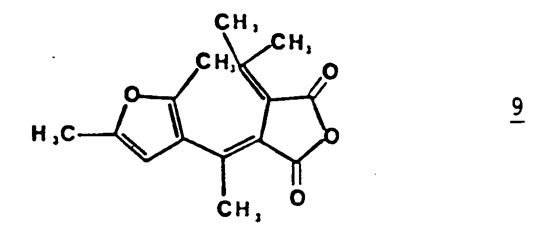 In Angew. Chem. 90, 927 (1978) werden u.a. ebenfalls Tetracyanoverbindungen erwähnt, und als Anwendung derartiger zweistufiger Redoxsysteme werden genannt: Redoxindikatoren, Elektronenakzeptoren, Katalysatoren für Elektronenübertragungen, lichtempfindliche Systeme und Elektronenleiter.
   Die in dieser Erfindung beschriebenen Furylidenmalononitrile können deshalb als wichtige neue Substanzklasse für elektro-optische Anwendungen bezeichnet werden.
7. Ein Vorteil von Furansubstituenten am Dihydroazulenrest ist auch die schnelle Rückreaktion der gefärbten Heptafulvenform nach Belichtung, die durch ziehende Substituenten für A, wie z.B. A = A¹ = CH=C(CN)₂, NO₂ zusätzlich begünstigt wird. Eine langsame Rückreaktion wird in Kirk-Othmer, Encyclopedia of chemical technology 3. Ed. Vol. 6, 124 (1979) ausdrücklich als Nachteil von organischen photochromen Materialien erwähnt. Für eine schnelle optische Informationsspeicherung bzw. -verarbeitung erfüllen somit Furylidenmalononitril-Derivate analog 5 ideale Voraussetzungen.
8. Ein weiterer Vorteil des Dihydroazulen-Heptafulven-Systems 3A ≷ 3B (R = OCH₃) ist auch seine relativ große Spektralverschiebung von etwa 380 nm auf etwa 470 nm mit einer gleichzeitigen Erhöhung des Extinktionskoeffizienten.

Verfahren zur Herstellung von 3- und/oder 4-substituierter HMF-Derivat sind z.B. beschrieben in Carbohydr. Res. 155, 99 (1986), wo über die Oxidation von 1,2; 4,5-Diisopropylidenfructose mit anschließender Grignard-Reaktion und Dehydratisierung verschiedene 3-substituierte HMF-Derivate z.B. der Formel 10 hergestellt werden. 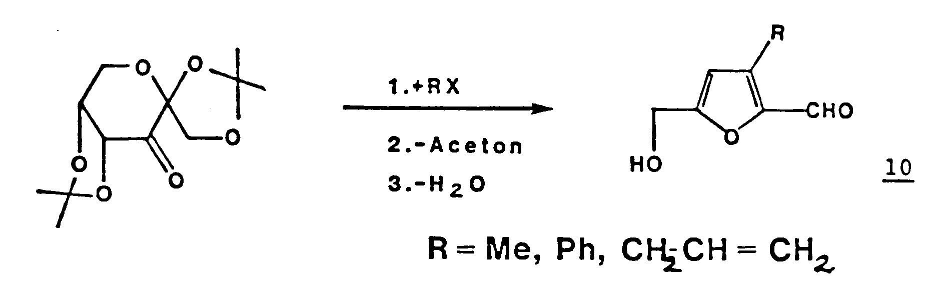
Andere Substituenten können nach den Regeln der Aromatenchemie in den Furanring eingeführt werden. Die 3- bzw. 4-substituierten HMF-Derivate werden wie das unsubstituierte HMF weiter umgesetzt.

Die Herstellung der Verbindungen mit n = 0 und
A = A₁ = CH₂OR¹
erfolgt durch eine Veretherung bzw. Veresterung z.B. mit Acetanhydrid von HMF mit anschließender Knoevenagel-Kondensation.

Für A = A₁ = 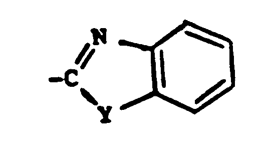
geht man von HMF und einem geeigneten ortho-disubstituierten Phenylderivat aus und stellt zuerst das Aminal bzw. O,N- oder S,N-Acetal her, das dann in einem Schritt z.B. mit BaMnO₄ zum Aromaten z.B. Benzimidazol und gleichzeitig zur Aldehydstufe (aus der Hydroxymethylgruppe) oxidiert wird.

Die gekreuzte Benzoinaddition mit HMF bzw. Derivaten wie z.B. 5-Acetoxymethylfurfural mit anschließender Oxidation führt zu Verbindungen z.B. der Formel 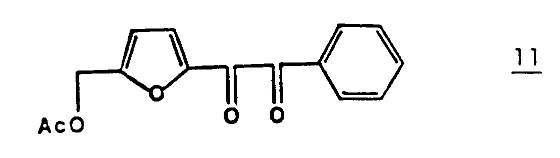
Die Oxidation der entacetylierten Verbindung und Knoevenagel-Kondensation mit Malonsäuredinitril führt zu Verbindungen gemäß der Formel 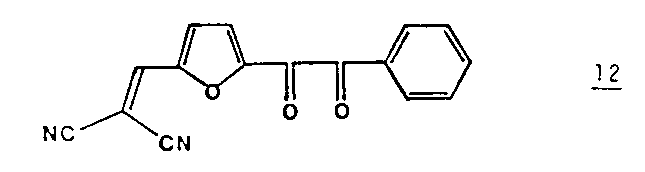
Allgemeine Herstellungsvorschrift für n = 1 und
A = A₂ = -CH=N--Z--N=CH-:
In einem geeigneten Lösungsmittel wird die Diaminoverbindung vorgelegt ggf. auch als Hydrochlorid oder ähnliches Salz, z.B. Hydrazin-Hydrochlorid oder -hydrogensulfat in Wasser, oder Ethylendiamin, 1,12-Diaminododecan oder p-Phenylendiamin in Methylenchlorid. Zu der gerührten Lösung wird bei Raumtemperatur die zweifache Molmenge HMF, gelöst in einem geeigneten Lösungsmittel, z.B. Wasser oder Methylenchlorid, langsam zugetropft. Entweder fällt nach kurzer Zeit ein Feststoff aus, der ggf. umkristallisiert wird, häufig jedoch direkt in der folgenden Oxidation eingesetzt werden kann, oder es muß zur Bildung der Schiffschen Base erhitzt und/oder wasserentziehende Mittel zugegeben werden. Die Bedingungen für die Bildung der Schiffscher Basen werden z.B. beschrieben in J. March, Advanced Organic Chemistry 3. Ed. 1985 J. Wiley & Sons, Inc., New York, S. 796 - 798.

Die Oxidation der beiden Hydroxymethylgruppen in den Amin-Hmf-Kondensationsprodukten kann beispielsweise mit aktivem Braunstein oder mit Bariummanganat durchgeführt werden. Im Gegensatz zu Aussagen der Literatur, Synthesis 1976, 133, wonach Azine in Gegenwart von Mangandioxid instabil wären, ist die Oxidation des HMF-Azins z.B. mit Braunstein möglich. Im erfindungsgemäßen Verfahren sollen aber neben den genannten Manganverbindungen andere Oxidationsmittel, die in der Lage sind, Hydroxymethylgruppen zur Aldehydstufe zu oxidieren, nicht ausgeschlossen werden. Dünnschichtchromatographisch kann der Oxidationsverlauf verfolgt werden. Die Oxidation kann in Benzol, Toluol oder auch Trichlorethan eventuell unter gleichzeitiger azeotroper Abdestillation des Reaktionswassers durchgeführt werden.

Die erhaltenen Dialdehyde werden dann nach den üblichen Verfahren ggf. mit TiCl₄-Katalyse mit Malonsäuredinitril umgesetzt und die dicyanvinylsubstituierten Furanderivate als gut kristallisierende Feststoffe isoliert.

Beispiele für Verbindungen mit n = 0
A = A₁ = CH₂OR¹:
- R¹ =: H, Methyl, Ethyl, n-Propyl, i-Propyl, n-Butyl, t-Butyl, Octyl, Phenyl, p-Hydroxyphenyl, p-Nitrophenyl, p-Dimethylaminophenyl, 2-Pyridyl, 1-Naphtyl, 2-Naphtyl, Trimethylsilyl, Triphenylsilyl, Acetyl, Palmitoyl, Benzoyl, p-Nitrobenzoyl, p-Dimethylaminobenzoyl, Methansulfonyl, p-Toluolsulfonyl, Phosphonyl (Dinatriumsalz), 2-Methoxyethyl, 4-Methoxybutyl,

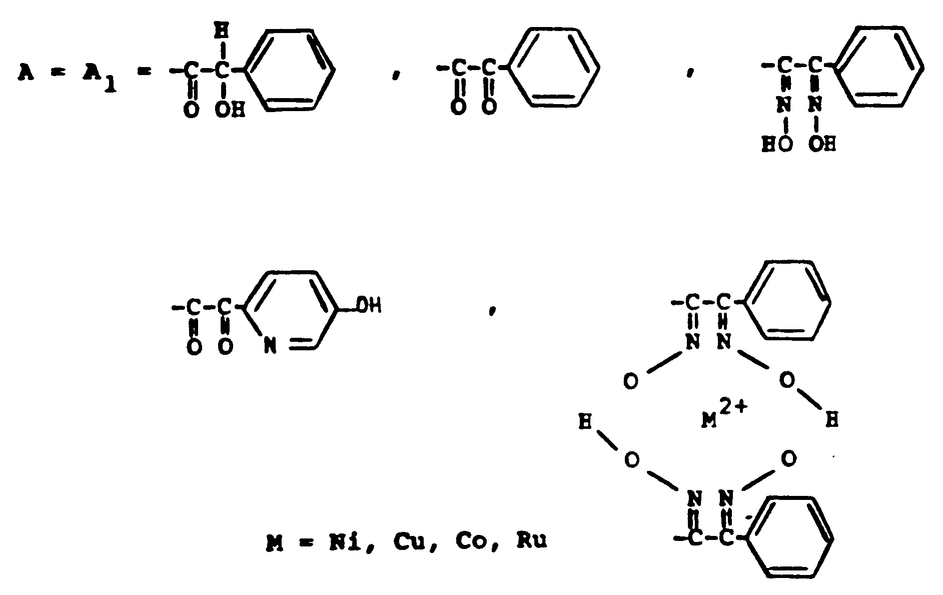

A = A₁ = CH=N-R³
- R³ =: Methyl, Butyl, Phenyl, p-Nitrophenyl, p-Dimethylaminophenyl
Die hier aufgeführten Beispiele sollen für diese Erfindung nur verdeutlichend und nicht beschränkend sein.

Beispiele für Verbindungen mit n = 1 und
A = A₂ = -CH=N-Z-N=CH-:
- Z =: Einfachbindung, 1,2-Ethandiyl, 1,4-Butandiyl, 1,12-Dodecandiyl, 1,4-Phenyldiyl, 1,3-Phenyldiyl, 1,4-Naphthalindiyl, 1,5-Naphthalindiyl, 1,8-Naphthalindiyl, 1,4-(2-Nitrophenyl)diyl, 3,5-(1,2,4-Triazol)diyl, 2,7-Fluorendiyl, 1,4-Anthrachinondiyl, 1,5-Antrachinondiyl, 2,6-Antrachinondiyl, 2,6-(4-Phenyl-1,3,5-triazin)diyl, 3,6-Acridindiyl, 2,6-Pyridindiyl, 3,7-(5-Phenothiazinium)diylchlorid, 3,8-(5-Ethyl-6-phenyl-phenanthridium)diylbromid,

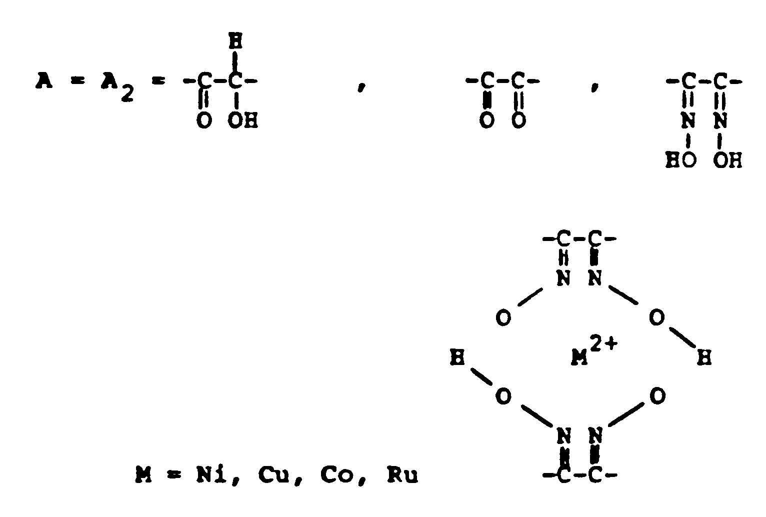

Die hier aufgeführten Beispiele sollen für diese Erfindung nur verdeutlichend und nicht beschränkend sein.

### Beispiel 1

### 2-(2′,2′-dicyanvinyl)-5-acetoxymethyl-furan

1,68 g (10 mmol) 5-Acetoxymethylfurfural und 0,66 g (10 mmol) Malonsäuredinitril werden in 50 ml Acetonitril gelöst und mit 40 mg β-Alanin und 10 Tropfen Essigsäure 10 Stunden zum Rückfluß erhitzt. Beim Abkühlen fällt der Katalysator aus und wird abfiltriert. Nach dem Einengen kristallisieren 2,2 g lila gefärbte Kristalle mit dem Schmelzpunkt 79°C; Umkristallisieren aus Methylenchlorid ergibt farblose Kristalle mit einem Schmelzpunkt von 80-82°C.
- IR (KBr):: 3130, 3100, 3040, 2990, 2940, 2230, 1750, 1610, 1565, 1225, 1195, 1005, 830 cm⁻¹
- ¹H-NMR(CDCl₃):: 7.48 ppm (s), 7.34 ppm (d, 3.7Hz), 6.67 ppm (d, 3.7Hz), 5.15 ppm (s), 2.13 ppm (s)

### Beispiel 2

### Bis-5,5′(2˝,2˝-dicyanvinyl)-furfural-azin

12,4 g (50 mmol) HMF-azin werden unter Erwärmen in 500 ml 1,1,2-Trichlorethan gelöst und nach Zugabe von 115,3 g Bariummanganat 7 Stunden zum Rückfluß erhitzt. Danach wird heiß filtriert und die Lösung langsam abgekühlt. Es entstehen gelb gefärbte nadelförmige Kristalle mit einem Schmelzpunkt von 214°C.
- IR (KBr):: 3140, 3115, 2850, 1670, 1630, 1490, 1410, 1250, 1175, 980, 955, 825, 795 cm⁻¹
- ¹H-NMR(DMSO):: 9.74 ppm (s,CHO), 8.71 ppm (s), 7.68 ppm (d, 3.7Hz), 7.38 ppm (d, 3.7Hz)
2,44 g (10 mmol) Dialdehyd und 1,32 g (20 mmol) Malonsäuredinitril werden in 150 ml Acetonitril heiß gelöst und nach Zugabe von 40 mg β-Alanin und 100 mg Essigsäure 4 Stunden zum Rückfluß erhitzt. Beim langsamen Abkühlen fallen Kristalle aus, die abfiltriert und gewaschen werden.
Schmelzpunkt 238-242°C
- IR (KBr):: 3120, 3030, 2220, 1595, 1535, 1380, 1285, 1205, 1197, 1135, 1023, 935, 810, 787, 770 cm⁻¹
- ¹H-NMR(DMSO):: 8.62 ppm, 8.55 ppm, 8.35 ppm, 7.65-7.25 ppm
- ¹³C-NMR(DMSO):: Signale des in größter Menge vorhandenen Rotameren:
154.0 ppm, 150.8 ppm, 150.0 ppm, 143.7 ppm, 125.7 ppm, 120.4 ppm, 114.2 ppm, 112.9 ppm, 77.9 ppm.

### Beispiel 3

### Bis 5-((2′,2′-dicyanvinyl)-furfuryl)-ether

4,70 g (20 mmol) Bis (5-formyl-furfuryl)-ether werden in 150 ml Toluol gelöst und mit 2,64 g (40 mmol) Malonsäuredinitril versetzt. Mit 50 mg β-Alanin und 200 mg Essigsäure wird 4 Stunden zum Rückfluß erhitzt und die Lösung langsam abgekühlt. Der Niederschlag wird abfiltriert und mit Toluol gewaschen (5,40 g Ausbeute). Schmelzpunkt 148°C (aus Toluol)
- IR (KBr):: 3130, 3045, 2920, 2224, 2212, 1608, 1548, 1494, 1340, 1211, 1194, 1140, 1123, 1029, 974, 798 cm⁻¹
- NMR (DMSO):: 8.26 ppm (s), 7.42 ppm (d, 3.65 Hz), 6.87 ppm (d, 3.65 Hz), 4.71 ppm (s)

### Beispiel 4

### Umsetzung von 2,2′-(2,5-furandiyldimethylidyne)bis-propandinitril mit 8-Methoxyheptafulven (1:1) in Dichlormethan

Zu einer frisch hergestellten, stickstoffgesättigten Lösung von 650 mg (4.45 mmol) 8-Methoxyheptafulven in 30 ml absolutem Dichlormethan werden 980 mg (4.80 mmol) 2,2′-(2,5-furandiyldimethylidyne)bis-propandinitril und eine Spatelspitze Hydrochinon gegeben und bei Raumtemperatur mit aufgesetztem Quecksilberventil unter Luftausschluß 18 h gerührt. Der Fortgang der Reaktion wird mittels Dünnschichtchromatogramm verfolgt. Nach beendeter Cycloaddition ist die ursprünglich tiefdunkelrote Lösung durchsichtig braun. Das Lösungsmittel wird abgezogen, das verbleibende braune Öl in Ether aufgenommen, filtriert, das Lösungsmittel im Vakuum abgezogen und der Rückstand im Hochvakuum getrocknet. Man erhält einen bräunlichen Feststoff.
Durch Säulenchromatographie über Kieselgel mit Dichlormethan/Petrolether 2:1 erhält man einen orangen Feststoff mit dem Schmelzpunkt 172-173°C in 52 %iger Ausbeute.
- MS(70 eV):: m/e = 322(100 %, M⁺, 295 (31 %, M-HCN)
- IR(KBr):: 3120, 3050, 2240, 1605, 1573, 1475, 1280, 1045, 820, 710 cm⁻¹
- UV/VIS(CH₃CN):: λₘₐₓ(1g ) = 250 (4.4), 327 (3.7), 443nm (4.6)
- ¹H-NMR(250MHz,CDCl₃):: δ = 3.81 (m;1H,H-8a),5.81 (dd,J= 10.4Hz,3.8Hz;1H,H-8), 6.32-6.38 (m;1H,- H-7), 6.52-6.65 (m;3H,H-4,H-5 H-6), 7.09 (d,J=4,0Hz;1H,Furan-H), 7.12 (s;1H,H-3), 7.30 (d,J=4.0Hz; 1H,-Furan-H), 7.46ppm(s;1H,Propandinitril-H)

### Beispiel 5

### Umsetzung von 2,2′-(2,5-Furandiyldimethylidyne)bis-propandinitril mit 8-Methoxyheptafulven (1:2) in Dichlormethan

Zu einer frisch hergestellten, stickstoffgesättigten Lösung von 1.80 g (13 mmol) 8-Methoxyheptafulven in 100 ml absolutem Dichlormethan werden 850 mg (3.86 mmol) 2,2′(2,5-furandiyldimethylidyne)bis-propandinitril und eine Spatelspitze Hydrochinon gegeben und bei Raumtemperatur mit aufgesetztem Quecksilberventil unter Luftausschluß 1 d gerührt. Der Fortgang der Reaktion wird mittels Dünnschichtchromatogramm verfolgt. Nach beendeter Cycloaddition ist die ursprünglich tiefrote Lösung durchsichtig braun. Das Lösungsmittel wird abgezogen, das verbleibende braune Öl in Ether aufgenommen, filtriert, das Lösungsmittel im Vakuum abgezogen und der Rückstand im Hochvakuum getrocknet. Man erhält einen bräunlichen Feststoff.

### Methanolabspaltung am entstandenen Tetrahydroazulen

Eine kräftig gerührte Lösung des entstandenen Tetrahydroazulens in 150 ml absolutem Benzol wird mit 20 g di-Phosphorpentoxid und 2.8 g Kaliumcarbonat versetzt und 6 h unter Rückfluß erhitzt. Nach Beendigung der Reaktion wird durch eine mit wenig Seesand und Kieselgel gefüllte Fritte filtriert. Das Filtrat wird im Vakuum eingeengt, man erhält ein gelb-braunes Öl.
Durch Chromatographie über Kieselgel mit Dichlormethan/Petrolether 1:1 erhält man einen orangen Feststoff mit dem Schmelzpunkt 182-184°C in 44 %iger Ausbeute.
- MS(70eV):: m/e = 424(100%,M⁺), 397(11%,M-HCN), 370(11%, M-2HCN)
- IR(KBr):: 1040, 780, 760, 698 cm⁻¹
- UV/VIS(CH₃CN):: λ ₘₐₓ(1gε) = 218(4.2), 245(4.2), 294(3.8), 354(4.0,s), 433,6(4.5), 456nm(4.4)
- ¹H-NMR(250MHz,CDCl₃):: δ = 3.78(m,2H,H-8a,H-8a′), 5.83(dd, J=10.2Hz,3.8Hz,2H,H-8,H-8′), 6.29-6.37 (m;2H,H-7,H-7′), 6.40(d, J=6.0Hz;2H,H-4,H-4′), 6.46-6.83(m, 4H,H-6,H-6′, H-5,H-5′), 6.86(s;2H, H-3,H-3′), 7.02 (s,2H,Furan-H)

## Patentansprüche

1. Dicyanvinylsubstituierte Furanderivate der allgemeinen Formel 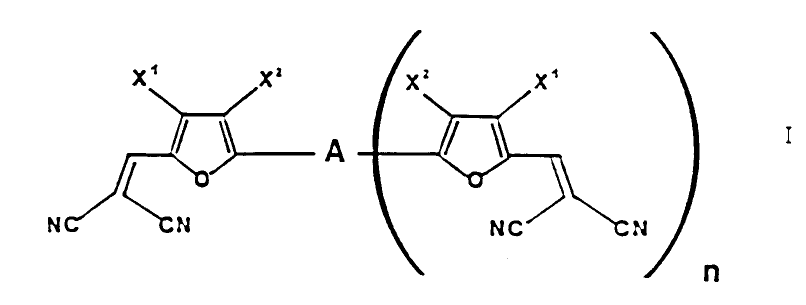 in der X¹ und X² gleich oder verschieden sind und für H, Me, Ph, CH₂-CH=CH₂, Halogen, NO₂, CN stehen, sowie im Falle von n = O:
A ist A₁ und CH₂OR¹ mit R¹ ist
a) H; Alkyl 1-8 C; Phenyl; ggf. in p-Stellung substituiert durch OH; NO₂ oder N(CH₃)₂; 2-Pyridyl; Naphtyl; Trimetylsilyl; Triphenylsilyl; Acetyl; Palmitoyl; Benzoyl; p-Nitropbenzoyl; p-Dimethylaminobenzoyl; Methansulfonyl; p-Toluolsulfonyl; Phosphonyl (Dinatriumsalz); 2-Methoxyethyl; 4-Methoxybutyl
b) [(CH₂)ₘ-O]ₚ-R², wobei
m = 1 bis 4
p = 1 bis 8 und
R² = R₁
c) 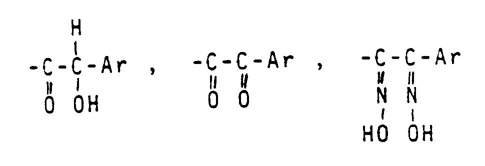 wobei
Ar = Phenyl, Naphthyl, Furyl, Thienyl, Pyrryl, Pyridyl
oder ein Metallkomplex davon,
d) CH=N-R³ mit R³ = Alkyl mit 1-4 C-Atomen, Phenyl, ggf. in p-Stellung substituiert durch NO₂, N(CH₃)₂,
oder
e) 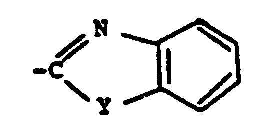 Y = NH, O, S ,
bedeutet,
und im Falle von n=1:
A ist A₂ und - CH=N-Z-N=CH- bedeutet, wobei
Z
a) eine Bindung,
b) ein geradkettig oder verzweigter, gesättigter oder ein- oder mehrfach ungesättigter Alkylrest mit 1-12 C-Atomen, 1,4-Phenyldiyl, 1,3-Phenyldiyl, 1,4-Naphthalindiyl, 1,5-Naphthalindiyl, 1,8-Naphthalindiyl, 1,4-(2-Nitrophenyl)diyl, 3,5-(1,2,4-Triazol)diyl, 2,7-Fluorendiyl, 1,4-Anthrachinondiyl, 1,5-Antrachinondiyl, 2,6-Antrachinondiyl, 2,6-(4-Phenyl-1,3,5-triazin)diyl, 3,6-Acridindiyl, 2,6-Pyridindiyl, 3,7-(5-Phenothiazinium)diylchlorid, 3,8-(5-Ethyl-6-phenyl-phenanthridium)diylbromid,
c) 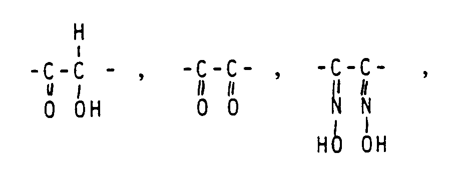 oder ein Metallkomplex davon
oder
d) -CH=CH- , -C≡C- , 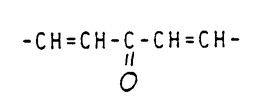 oder -CH₂-O-CH₂-
ist.

2. Verfahren zur Herstellung der Verbindungen nach Anspruch 1, dadurch gekennzeichnet, daß im Falle von n = 0 die Hydroxymethylgruppe in einem ggf. substituierten 5-Hydroxymethylfurfural derivatisiert, z.B. verethert oder verestert wird und dann die Aldehydgruppe nach Knoevenagel mit Malonsäuredinitril umgesetzt wird oder die Aldehydgruppe zuerst derivatisiert wird, z.B. Schiffsche Base, Aminal, gekreuzte Benzoinaddition, anschließend die Hydroxymethylgruppe oxidiert und dann nach Knoevenagel mit Malonsäuredinitril umgesetzt wird.

3. Verfahren zur Herstellung der Verbindungen nach Anspruch 1, dadurch gekennzeichnet, daß im Falle von n = 1 zwei ggf. substituierte 5-Hydroxymethylfurfural-Moleküle zuerst mit einem, mindestens zwei NH₂-Gruppen enthaltenden, Molekül kondensiert werden und die beiden Hydroxymethylgruppen am Furanring oxidiert und nach Knoevenagel mit Malonsäuredinitril umgesetzt werden oder durch intermolekulare Wasserabspaltung an den Hydroxymethylgruppen zwei Hydroxymethylfurfuralmoleküle verknüpft werden und die beiden Aldehydfunktionen nach Knoevenagel mit Malonsäurediitril umgesetzt werden oder in einer Benzoinaddition zwei ggf. substituierte Hydroxymethylfurfurale über eine zwei Kohlenstoffatome umfassende Kette verknüpft werden, die Hydroxymethylgruppen am Furanring oxidiert und nach Knoevenagel mit Malonsäuredinitril umgesetzt werden.

4. Verwendung der Verbindungen nach den Ansprüchen 1 bis 3 sowie derjenigen mit n = 0, und A₁ = NO₂, -CH=C(CN)₂ als 2π-Komponenten für Cycloadditionsreaktionen.

5. Verwendung der Verbindungen nach den Ansprüchen 1 bis 4 als Reaktionspartner für (8+2)-Cycloadditionen mit 8-Methoxyheptafulven.

6. Verwendung der Cycloaddukte nach den Ansprüchen 1 bis 5, die nach Methanoleliminierung als lichtsensitive Dihydroazulene, die reversibel mit Heptafulvenstrukturen im Gleichgewicht stehen, vorliegen, als chromogene Stoffe.

7. Verwendung der Verbindungen nach den Ansprüchen 1 bis 6 zur Herstellung von optischen Datenaufzeichnungssystemen, wie Displays, Projektionsbildschirme, Datensichtgeräte und Datenanzeigegeräte.

8. Verwendung der Verbindungen nach den Ansprüchen 1 bis 6 zur reversiblen Elektronenaufnahme.

9. Verwendung der Verbindungen nach den Ansprüchen 1 bis 6 zur Herstellung von organischen, elektrischleitenden und magnetischen bzw. ferromagnetischen Stoffen.

10. Verwendung der Verbindungen nach den Ansprüchen 1 bis 6 zur Herstellung von Elektronen-Transfer-Katalysatoren bzw. Elektronen-Transfer-Vermittlern.

## Claims

1. Dicyanvinyl-substituted furan derivatives of the general formula 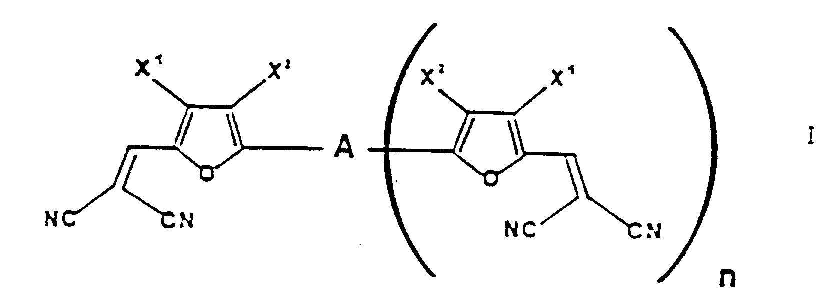 in which X¹ and X² are the same or different and stand for H, Me, Ph, CH₂-CH=CH₂, halogen, NO₂, CN, and in the case of n = 0:
A is A₁ and CH₂OR¹ where R¹ is
a) H; alkyl 1-8 C; phenyl; possibly in the p-position substituted by OH; NO₂ or N(CH₃)₂; 2-pyridyl; naphthyl; trimethylsilyl; triphenylsilyl; acetyl; palmitoyl; benzoyl; p-nitrobenzoyl; p-dimethylaminobenzoyl; methane sulfonyl; p-toluene sulfonyl, phosphonyl (disodium salt); 2-methoxyethyl; 4-methoxybutyl
b) [(CH₂)ₘ-O]ₚ-R², wherein
m = 1 to 4
p = 1 to 8 and
R² = R₁
c) 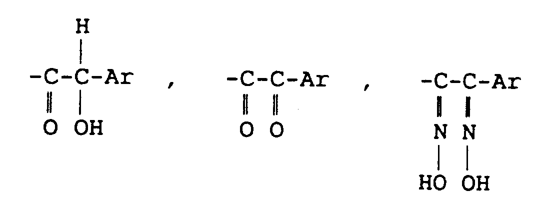 wherein
Ar = phenyl, naphthyl, furyl, thienyl, pyrryl, pyridyl
or a metal complex thereof,
d) CH=N-R³ where R³ = alkyl with 1-4 C-atoms; phenyl, possibly in the p-position substituted by NO₂, N(CH₃)₂,
or
e) 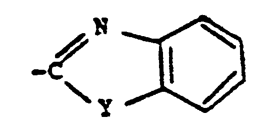 Y = NH, O, S
and in the case of n=1:
A is A₂ and signifies - CH=N-Z-N=CH-, wherein
Z is
a) a bond,
b) an alkyl residue, straight-chained or branched, saturated or mono-unsaturated or poly-unsaturated, with 1-12 C-atoms, 1,4-phenyldiyl, 1,3-phenyldiyl, 1,4-naphthalindiyl, 1,5-naphthalindiyl, 1,8-naphthalindiyl, 1,4-(2-nitrophenyl)diyl, 3,5-(1,2,4-triazole)diyl, 2,7-fluorenediyl, 1,4-anthraquinonediyl, 1,5-anthraquinonediyl, 2,6-anthraquinonediyl, 2,6-(4-phenyl-1,3,5-triazine)diyl, 3,6-acridinediyl, 2,6-pyridinediyl, 3,7-(5-phenothiazinium)diylchloride, 3,8-(5-ethyl-6-phenyl-phenanthridium)-diylbromide,
c) 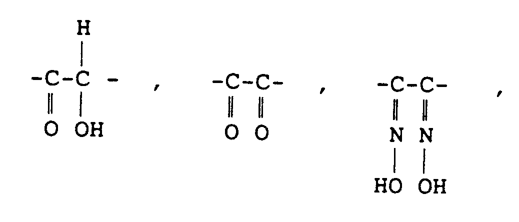 or a metal complex thereof
or
d) -CH=CH- , -C≡C-, 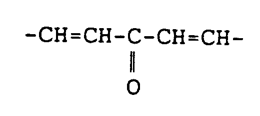 or -CH₂-O-CH₂- .

2. Process for the manufacture of the compounds according to claim 1, characterized in that in the case of n = 0, the hydroxymethyl group is derived from a possibly substituted 5-hydroxymethylfurfural, for example is etherified or esterified and then the aldehyde group is caused to react according to Knoevenagel with malonic acid dinitrile or the aldehyde group is first of all derived, for example Schiff's base, aminal, crossed benzoin addition, subsequently the hydroxymethyl group is oxidized and then caused to react according to Knoevenagel with malonic acid dinitrile.

3. Process for the manufacture of the compounds according to claim 1, characterized in that in the case of n = 1, two possibly substituted 5-hydroxymethylfurfural molecules are first of all condensed with a molecule containing at least two NH₂ groups, and the two hydroxymethyl groups are oxidized at the furan ring and are caused to react according to Knoevenagel with malonic acid dinitrile or by means of intermolecular splitting-off of water at the hydroxymethyl groups two hydroxymethylfurfural molecules are connected and the two aldehyde functions are caused to react according to Knoevenagel with malonic acid dinitrile or in a benzoin addition two possibly substituted hydroxymethylfurfurals are connected by way of a chain comprising two carbon atoms, the hydroxymethyl groups at the furan ring are oxidized and are caused to react according to Knoevenagel with malonic acid dinitrile.

4. Use of the compounds according to claims 1 to 3 as well as those with n = 0, and A₁ = NO₂, -CH=C(CN)₂ as 2π-components for cycloaddition reactions.

5. Use of the compounds according to claims 1 to 4 as reaction partners for (8+2)-cycloadditions with 8-methoxyheptafulvene.

6. Use of the cycloadducts according to claims 1 to 5, which after methanol elimination exist as light-sensitive dihydroasulenes, which are in equilibrium in a reversible manner with heptafulvene structures, as chromogenic substances.

7. Use of the compounds according to claims 1 to 6 for the manufacture of optical data recording systems, like displays, projection screens, data visual units and data display units.

8. Use of the compounds according to claims 1 to 6 for reversible electronic recording.

9. Use of the compounds according to claims 1 to 6 for the manufacture of organic, electrically conductive and magnetic or ferromagnetic substances.

10. Use of the compounds according to claims 1 to 6 for the manufacture of electron transfer catalysts or electron transfer agents.

## Revendications

1. Dérivés de furane substitués en dicyanovinyle, de formule générale 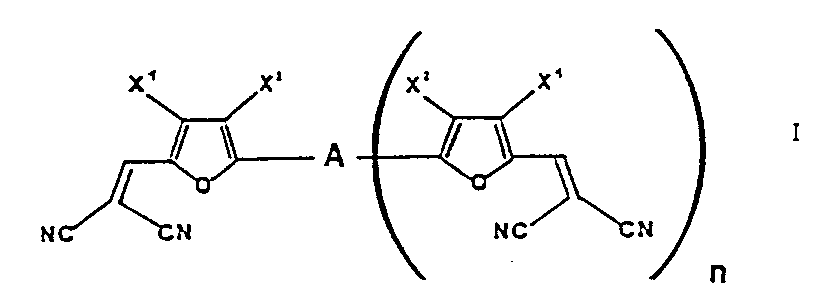 dans laquelle X¹ et X² sont égaux ou différents et représentent H, Me, Ph, CH₂-CH=CH₂, un halogène, NO₂, CN, ainsi que dans le cas de n = 0 :
A est A₁ et CH₂ OR¹, R¹ étant
a) H; alkyl contenant de 1 à 8 atomes de C; phényl, le cas échéant substitué en position p par un radical OH; NO₂ ou N(CH₃)₂; 2-pyridyl; naphtyl; trimetylsilyl; triphenylsilyl; acetyl; palmitoyl; benzoyl; p-nitropbenzoyl; p-diméthylaminobenzoyl; methansulfonyl; p-toluolsulfonyl; phophonyl (sel de disodium), 2-methoxyéthyl, 4-méthoxybutyl
b) [(CH₂)_{m-O}]p-R², où
m = 1 à 4
p = 1 à 8 et
R²= R₁
c) 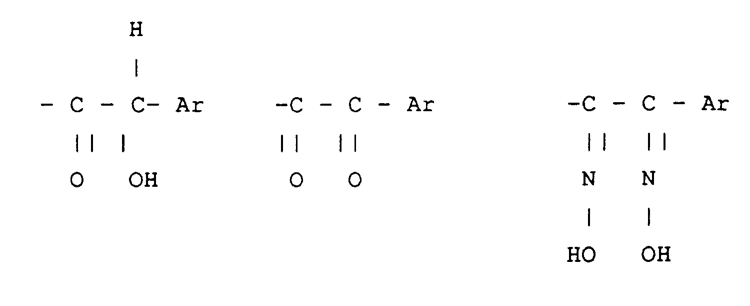 où
Ar = phenyl, naphtyl, furyl, thiényl, pyrryl, pyridyl ou un complexe métallique de ceux-ci,
d) CH=N-R³ avec R³ = alkyl avec 1 à 4 atomes de carbone; phényl, le cas échéant substitué en position p par NO₂, N(CH₃)₂,
ou
e) 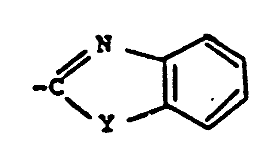 Y = NH, O, S
et,
dans le cas de n = 1 :
A est A₂ et signifie CH=N-Z-N=CH- où,
Z est
a) une liaison
b) un résidu alkyl avec 1 à 12 atomes de carbone, à chaîne droite ou ramifiée, saturé ou une où plusieurs fois insaturé, 1,4-phényldyil, 1,3-phényldiyl, 1,4-naphthalin-diyl, 1,5-naphthalindiyl, 1,8-naphthalindiyl, 1,4-(2-nitrophényl)diyl, 3,5-(1,2,4-triazol)diyl, 2,7-fluoren-diyl, 1,4-anthrachinondiyl, 1,5-antrachiondiyl, 2,6-antrachinondiyl, 2,6-(-4-phényl-1,3,5-triazin)diyl, 3,6-acridindiyl, 2,6-pyridindiyl, 3,7-(5-phenothiazinium) diylchorure, 3,8-(5-éthyl-6-phénylphenanthridium) diylbromure
c) 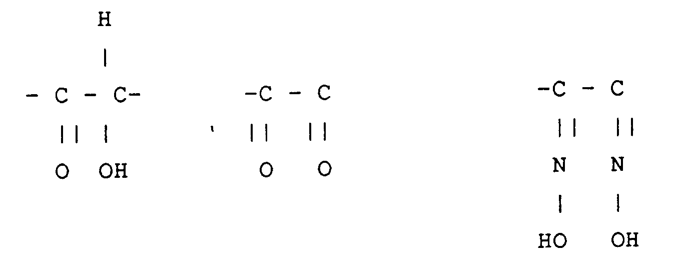
ou un complexe métallique de celui-ci ou
d) -CH=CH-, -C≡C-, 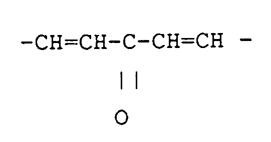 ou -CH₂-O-CH₂-.

2. Procédé de préparation des combinaisons selon la revendication 1, caractérisé en ce que, dans le cas ou n = 0, le groupe hydroxyméthyl est déviré pour donner un 5-hydroxyméthylfurfural, le cas echéant substitué, par exemple éthérisé ou estérisé, et le groupe aldéhyde étant converti selon Knoevenagel avec du dinitrile d'acide malonique ou le groupe aldéhyde est d'abord dérivé, par exemple avec une base de Schiff, un aminal, une addition croisée de benzoïne, puis le groupe hydroxyméthyl est oxydé et ensuite converti selon Knoevenagel avec du dinitrile d'acide malonique.

3. Procédé de préparation des combinaisons selon la revendication 1, caractérisé en ce que, dans le cas ou n = 1, on condense d'abord deux molécules de 5-hydroxyméthylfurfural, le cas échéant substituées, avec une molécule contenant au moins deux groupes NH₂, et les deux groupes hydroxyméthyl situés sur l'anneau furane étant oxydés et convertis selon Knoevenagel, avec du dinitrile d'acide malonique ou combinés par dissociation intermoléculaire de l'eau sur les groupes hydroxyméthyle de deux molécules d'hydroxyméthylefurfural, et les fonctions aldéhyde étant converties selon Knoevenagel avec du dinitrile d'acide malonique ou, combinées avec une addition de benzoïne pour donner deux hydroxyméthylfurfurales le cas échéant substitué, par l'intermédiaire d'une chaîne comprenant deux atomes de carbone, les groupes hydroxyméthyle situés sur l'anneau furfurane étant oxydés et convertis selon Knoevenagel avec du dinitrile d'acide malonique.

4. Utilisation des combinaisons selon les revendications 1 à 3, ainsi que celles données par n = 0, et A₁ = NO₂, CH=C(CN)₂ à titre de composant 2π pour des réactions de cycloaddition.

5. Utilisation des combinaisons selon les revendications 1 à 4, comme partenaire de réaction pour des cycloadditions de type (8 + 2) avec des 8-methoxyheptafulves.

6. Utilisation de cycloadditifs selon les revendications 1 à 5, se présentant après l'élimination méthanol à titre de dihydroazulène, sensible à la lumière, placé en équilibre réversible avec les structures heptafulves, à titre de substance chromogène.

7. Utilisation des combinaisons selon la revendication 1 à 6, pour la préparation de systèmes d'enregistrement de données optiques, tels que des écrans d'affichage, des écrans de projection, des appareils de visualisation de données et des appareils d'affichage de données.

8. Utilisation des combinaisons selon la revendication 1 à 6, pour l'enregistrement réversible des électrons,

9. Utilisation des combinaisons selon la revendication 1 à 6, pour la fabrication de substances organiques conductrices de l'électricité et magnétiques respectivement férromagnétiques.

10. Utilisation des combinaisons selon les revendications 1 à 6, pour la fabrication de catalyseurs d'accélérateurs d'électrons par transfert.
